**Europäisches Patentamt**

**European Patent Office**

**Office européen des brevets**

(19)

(11) Veröffentlichungsnummer: **0 242 510**
**A2**

(12) ## EUROPÄISCHE PATENTANMELDUNG

(21) Anmeldenummer: **87101230.8**

(22) Anmeldetag: **29.01.87**

(51) Int. Cl.³: **A 61 N 1/362**

(30) Priorität: **19.04.86 DE 3613283**

(43) Veröffentlichungstag der Anmeldung:
**28.10.87 Patentblatt 87/44**

(84) Benannte Vertragsstaaten:
**AT BE CH DE ES FR GB IT LI NL SE**

(71) Anmelder: **Osypka, Peter, Dr. Ing.**
**Basler Strasse 109**
**D-7889 Grenzach-Wyhlen(DE)**

(72) Erfinder: **Osypka, Peter, Dr. Ing.**
**Basler Strasse 109**
**D-7889 Grenzach-Wyhlen(DE)**

(74) Vertreter: **Schmitt, Hans, Dipl.-Ing. et al,**
**Patentanwälte Dipl.-Ing H. Schmitt Dipl.-Ing. W. Maucher**
**Dreikönigstrasse 13**
**D-7800 Freiburg(DE)**

(54) **Herzschrittmacher mit einem Steuerteil zur Erhöhung der Herz-Ruhefrequenz.**

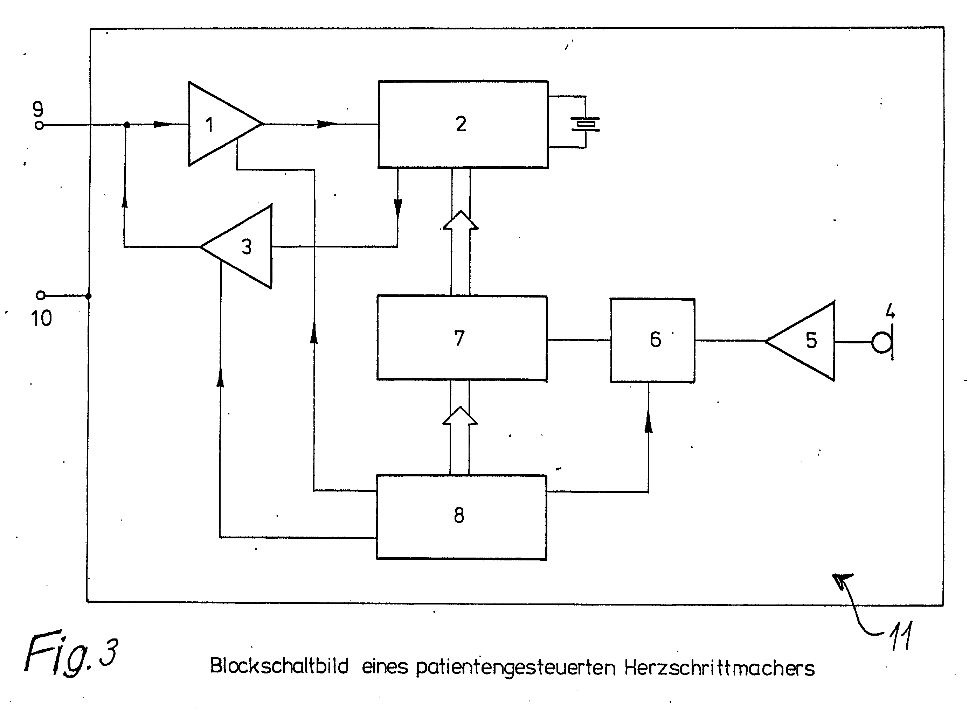

(57) Ein Herzschrittmacher (11) ist dadurch bezüglich seiner Stimulationsfrequenz veränderbar und somit an erhöhte körperliche Leistungen des Herzschrittmacher-Patienten anpaßbar, daß er ein mechanisch von außen durch Klopfen bedienbares Betätigungsorgan (4) zur Erhöhung der Herz-Ruhefrequenz und zur Zurückschaltung auf die Herz-Ruhefrequenz aufweist. Meßfühler zum Messen von Sauerstoffkonzentrationen des Blutes, zur Messung der Bluttemperatur, zum Messen der Atemfrequenz und dgl., die störanfällig und bezüglich ihrer Langzeitwirkung noch nicht genügend geprüft sind sowie in den meisten Fällen zusätzliche Energie benötigen und das Operationsrisiko und den Operationsaufwand erhöhen, können dadurch vermieden werden (Figur 3).

EP 0 242 510 A2

*Fig.3*  Blockschaltbild eines patientengesteuerten Herzschrittmachers

DIPL.-ING. H. SCHMITT
DIPL.-ING. W. MAUCHER

78 FREIBURG I. BR
DREIKÖNIGSTR. 13
TELEFON: (0761) 78753

0242510

Herr
Dr.Ing.Peter Osypka
Basler Straße 109
7889 Grenzach-Wyhlen

UNSERE AKTE · BITTE STETS ANGEBEN:

E 87 128 MR

**Herzschrittmacher mit einem Steuerteil**
**zur Erhöhung der Herz-Ruhefrequenz**

Die Erfindung betrifft einen Herzschrittmacher mit einem
Steuerteil zur Erhöhung der Herz-Ruhefrequenz bei erhöhter
körperlicher Leistung des Herzschrittmacherpatienten, wobei der Herzschrittmacher ein mechanisch von außen durch
die Haut bedienbares Betätigungsorgan zur Erhöhung der Herz-
Ruhefrequenz und zur Zurückschaltung auf die Herz-Ruhefrequenz aufweist.

Ein derartiger Herzschrittmacher ist aus der US-PS 3 945 387
bekannt. Dabei beschreibt diese Druckschrift in erster
Linie eine Lösung, bei welcher der Herzschrittmacher magnetisch oder elektromagnetisch bedienbare Schalter aufweist,
bei denen entsprechende Schaltkontakte betätigt werden sollen. Diese Druckschrift erwähnt auch die Möglichkeit, diese
Schalter durch physikalischen Druck von außen auf den Körper zu betätigen, ohne dazu aber nähere Angaben zu machen.
Es muß jedoch als ungünstig angesehen werden, wenn ein
Herzschrittmacher-Besitzer zur Änderung der Herz-Ruhefrequenz, beispielsweise zur Erhöhung dieser Frequenz bei Kraftanstrengungen oder Belastungen zunächst einen ausreichend
großen physikalischen Druck auf seinen Körper ausüben muß,

/2

um einen Schalter zu betätigen. Vor allem muß befürchtet werden, daß dies bei mehrfacher Betätigung wegen der entsprechenden Belastung des Herzschrittmachers und der von ihm ausgehenden Elektrodenanordnung zu mechanischen Überbelastungen und Beschädigungen führt. Außerdem kann ein solcher wiederholter physikalischer Druck auf dem Körper diesen selbst reizen und gar zur Schmerzen führen.

Auch aus der DE-OS 20 48 612 ist ein sogenannter Bedarfsschrittmacher bekannt, der im Gegensatz zu einem kontinuierlich arbeitenden Herzschrittmacher einen Impuls nur nach einer vorbestimmten Zeit nach dem Auftreten des letzten natürlichen Herzschlages erzeugt. Dabei beschreibt diese Vorveröffentlichung einen Schrittmacher mit variabler Rate, dessen Betriebsrate nicht durch mechanischen Stoß oder Vibration beeinflußt werden soll und der einen monostabilen magnetischen Zungenschalter aufweist, der durch ein magnetisches Feld in der Nähe der Brust des Patienten betätigt werden soll. Es ist also für die Betätigung dieses Zungenschalters ein spezielles Gerät erforderlich.

Aus der DE-OS 25 20 387 ist eine Prüfeinrichtung für künstliche Schrittmacher bekannt, mit welcher dem Herzschrittmacher von außen Energie zugeführt wird, damit der Herzschrittmacher selbst für seine Prüfung genügend Energie abgeben kann, die er normalerweise nicht abgibt. Eine Änderung der Stimulationsfrequenz ist dabei nicht vorgesehen.

Nicht zuletzt wegen der erwähnten Schwierigkeiten bei einem mechanischen Druck auf den Körper des Patienten oder wegen der Notwendigkeit spezieller von außen anzuwendender Geräte gibt es auch bereits ein langjähriges und vielfältiges Bemühen der Fachwelt, einen Herzschrittmacher zu schaffen, der ohne einen solchen Druck auf den Patienten oder ohne spezielle Geräte eine erhöhte Stimulation bei Kraftanstrengungen oder Belastungen des Trägers des Herzschrittmachers erlaubt.

Aus der DE-PS 27 17 659 ist ein derartiger Herzschrittmacher bekannt, bei welchem eine Meßsonde die Veränderung des venösen Blutsauerstoff-Sättigungswertes erfaßt und auf einen Herzschrittmacher überträgt, der dadurch zu entsprechenden Frequenzen angeregt werden soll. Dabei geht aus der DE-PS 27 17 659 schon ein vielfältiges Bemühen der Fachwelt hervor, sich ändernde Blutwerte bei Belastung des Benutzers zu messen und zur Steuerung der Herzschrittmacherfrequenz zu verwenden.

Aus der DE-OS 34 22 913 ist eine Regelschaltung zur Anpassung der Stimulationsfrequenz eines Herzschrittmachers an die Belastung eines Patienten bekannt, bei welcher ebenfalls die Blutsauerstoffsättigung gemessen und die Meßgrösse als Regelgröße für die Herzschrittmacher-Stimulationsfrequenz verwendet werden soll.

Auch die DE-OS 34 29 596 ist ein Beispiel dafür, eine Sauerstoff-Meßelektrode vorzusehen, um eine physiologische Frequenzsteuerung eines Herzschrittmachers durchführen zu können.

Aus der DE-PS 34 19 439 ist ein Herzschrittmacher mit einer Steuervorrichtung zur automatischen Anpassung der Stimulationsfolgefrequenz an die Belastung des Patienten mit einem Temperaturfühler zum Erfassen der Temperatur des venösen Blutes im Herzen bekannt. Somit ist auch in diesem Falle eine entsprechende Meßvorrichtung und deren Verbindung mit dem Herzschrittmacher erforderlich.

Aus der DE-OS 34 28 975 ist ein Vorschlag bekannt, einen Herzschrittmacher über die Atmung des Benutzers zu steuern und abhängig von der Atmung die Stimulationsfrequenz zu verändern.

Die vorstehenden Literaturstellen sind dabei nur Beispiele und enthalten ihrerseits umfangreiche Hinweise auf weitere Literaturstellen, die sich mit Frequenzadaptierung an die Belastung des Patienten befassen. Dabei ist all diesen Vorschlägen gemeinsam, daß ein separater, zu einer Meßstelle im Körper führender, dort zu verankernder Meßfühler erforderlich ist, der somit ebenfalls implantiert werden muß. Dabei ist die Langlebigkeit solcher Meßfühler innerhalb des menschlichen Körpers noch nicht oder nicht ausreichend erprobt. Darüberhinaus muß jeder derartige Meßfühler über einen separaten Stecker elektrisch mit dem Herzschrittmacher verbunden werden. Dadurch wird das gesamte System sehr komplex, insbesondere was die Dichtigkeit und auch eventuelle Reparaturen bei Brüchen der Zuleitung betrifft.

Ferner benötigen solche Meßfühler in der Regel zusätzliche Energie, die auch dann von der Batterie des Herzschrittmachers abgezogen werden muß, wenn diese Energie relativ gering ist. In jedem Falle bewirkt dies eine Verkürzung der Lebensdauer der Energiequelle des Gesamtsystemes.

Ferner ist noch nicht zu übersehen, wie die mechanische Beanspruchung durch die Körperbewegung sich auf diese Meßfühler und Sensoren auswirken wird. Auch die Langzeitstabilität der elektrischen Werte der Sensoren liegt noch keineswegs mit genügender Sicherheit fest, so daß nicht auszuschließen ist, daß bei solchen Systemen in der Praxis spätere Nachkalibrierungen erforderlich werden.

Auf der anderen Seite zeigt das große Bemühen der Fachwelt um die Lösung dieses Problemes, daß ein wirkliches Bedürfnis an Herzschrittmachern mit veränderbarer Stimulationsfrequenz besteht, wobei die Entwicklung eindeutig von mehr oder weniger mechanischen Schaltern, die durch einen physikalischen Druck auf den Körper des Patienten oder bevorzugt mittels spezieller Geräte zur magnetischen oder elektromagnetischen Betätigung arbeiteten, zu solchen Geräten führt, die aufgrund von Messungen innerhalb des Körpers des Patienten betätigt werden sollen, aber die vorbeschriebenen Probleme bewirken.

Es besteht deshalb die Aufgabe, einen Herzschrittmacher der eingangs erwähnten Art zu schaffen, bei welchem die Stimulationsfrequenz für erhöhte Aktivitäten des Patienten vergrößert werden können, ohne daß aufwendige, störanfällige und bzgl. ihrer Wirkungsweise nicht sichere Meßfühler od.dgl. Sensoren oder spezielle, von außen her anzuwendende Betätigungsgeräte erforderlich sind und wobei der Benutzer auf einfache und bequeme Weise die Änderung der Stimulationsfrequenz selbst herbeiführen kann.

Die überraschende und in vorteilhafter Weise auch relativ einfach zu realisierende Lösung dieser scheinbar widersprüchlichen Aufgabe besteht bei einem Herzschrittmacher der eingangs erwähnten Art darin, daß als Betätigungsorgan ein klopfempfindlicher Schalter oder Sensor vorgesehen ist und zum Auslösen oder Betätigen wenigstens ein Klopfimpuls erforderlich ist.

Entgegen der neueren Entwicklung, Meßsonden, Meßfühler und Sensoren vorzusehen, um bei erhöhter Aktivität sich ändernde körpereigene Werte festzustellen und diese als Regelgrößen in dem Herzschrittmacher zu benutzen geht also die Erfindung von einer möglichst angeglichenen Imitation natürlicher Vorgänge bei der Erhöhung der Herzfrequenz ab. Andererseits erlaubt sie aber auch den Verzicht auf von außen her zu betätigende Geräte, ohne dem Patienten einen für den Herzschrittmacher gefährlichen und eventuell auch schmerzhaften Druck zur Betätigung von Schaltkontakten zuzumuten.

Es werden also sowohl Störfaktoren durch Meßfühler und dgl. von vorneherein als auch spezielle Betätigungsgeräte oder Unbequemlichkeiten durch einen physikalischen Druck auf den Bereich des Herzschrittmachers vermieden.

Der Patient braucht lediglich vor Beginn einer erhöhten Aktivität gegen das Betätigungsorgan bzw. den Bereich von dessen Implantation zu klopfen, um eine erhöhte, vorher einprogrammierte Stimulationsfrequenz auszulösen. Dies stellt eine sehr einfach und bequem durchführbare Manipulation dar, die ohne Fremdeinwirkung, ohne fremde Geräte und dgl. und auch ohne Gefahr für die Herzschrittmacheranordnung durchgeführt werden kann. Ebenso kann er nach der Beendigung der Aktivität wieder auf die Herz-Ruhefrequenz zurückschalten. Dadurch ergibt sich der weitere Vorteil, daß beim Implantieren eines solchen Herzschrittmachers der Aufwand gegenüber nicht anpaßbaren Herzschrittmachern nicht vergrößert wird. Darüberhinaus wird auch die Schaltung innerhalb des Herzschrittmachers relativ einfach und die Erhöhung oder Verminderung der Stimulationsfrequenz bedarf keiner Energie aus der Batterie des Herzschrittmachers.

Da das Auslösen und Betätigen durch Klopfimpuls erfolgen kann, können auch sonstige Druckbelastungen, denen der Träger des Herzschrittmachers unter Umständen ungewollt einmal ausgesetzt sein könnte, keine Änderung der Stimulationsfrequenz auslösen.

Eine Ausführungsform der Erfindung kann dabei darin bestehen, daß der klopfempfindliche Sensor einen piezo-elektrischen Kristall od.dgl. enthält. Damit läßt sich ein- oder auch mehrmaliges Klopfen besonders gut in ein elektrisches und damit innerhalb einer Steuerschaltung eines Herzschrittmachers verwertbares Signal umsetzen.

Die Zahl der mechanischen Einwirkungen auf den Sensor zur Ansteuerung der verschiedenen einprogrammierten Frequenzen des Herzschrittmachers kann verschieden sein, so daß auch mehrere unterschiedliche Stimulationsfrequenzen einstellbar sein können.

Eine weitere Ausgestaltungsmöglichkeit der Erfindung kann darin bestehen, daß der Sensor zur mechanischen Auslösung einer Frequenzänderung des Herzschrittmachers ein derart unempfindliches Mikrophon ist, daß es nur auf Klopfen reagiert.

Weitere Ausgestaltungsmöglichkeiten der Erfindung sind Gegenstand der Ansprüche 4 u.6 bis 12.

Besonders zweckmäßig ist dabei die Maßnahme nach Anspruch 7, in welchem eine konkrete Möglichkeit über die Einstellung der Ansprechschwelle angegeben ist, durch welche unbeabsichtigte Betätigungen von vorneherein vermieden werden können.

Auch Anspruch 8 ist von erheblicher Bedeutung, weil darin eine Möglichkeit angegeben wird, wie der Anstieg oder auch der Abfall von einer auf die andere Stimulationsfrequenz zeitlich gestreckt werden kann, was jeweils auf den Benutzer abgestimmt sein kann.

Ebenfalls wichtig ist der Gegenstand des Anspruches 9, der eine Möglichkeit vorsieht, die Herz-Ruhefrequenz nach einer

/8

bestimmten vorgegebenen Zeit selbsttätig einzuschalten, um zu verhindern, daß ein vergeßlicher Patient zu lange mit einer zu hohen Frequenz stimuliert wird. Sollte der Patient oder Benutzer eine längere Dauer der erhöhten Stimulationsfrequenz wünschen, kann er nach Beendigung dieser Höchstzeit durch ein erneutes Klopfsignal auf ganz einfache Weise wieder die erhöhte Frequenz auslösen.

Insgesamt ergibt sich ein Herzschrittmacher einfacher Bauart, der sehr robust ist und dennoch dem Patienten eine Anpassung der Stimulationsfrequenz an erhöhte Belastungen erlaubt, ohne gefährliche oder schmerzhafte physikalische Druckbelastungen auf den Patienten oder spezielle, von außen anwendbare Betätigungsgeräte oder aber zusätzlich implantierbare Meßfühler zu benötigen. Die Erfindung macht sich dabei den Gedanken zunutze, daß für bestimmte Aktivitäten gewisse Herzfrequenzen ausreichend sind und für jeden Patienten eine individuelle Einstellung der verschiedenen auslösbaren Frequenzstufen genügt, um den jeweiligen individuellen Bedürfnissen gerecht werden zu können, wobei gleichzeitig Wert auf eine sehr einfache und ungefährliche Bedienbarkeit gelegt ist und Fehlbedienungen oder ungewollte Auslösungen praktisch ausgeschlossen sind.

Nachstehend ist die Erfindung mit ihren ihr als wesentlich zugehörenden Einzelheiten anhand der Zeichnung noch näher beschrieben.

Es zeigt:

Fig. 1 eine Seitenansicht eines Herzschrittmachers mit einem durch die Haut mechanisch betätigbaren Sensor am Gehäuse,

Fig. 2 eine der Fig.1 entsprechende Darstellung eines Herzschrittmachers, bei welchem ein mechanisch durch die Haut betätigbarer Sensor oder Schalter über eine Leitung mit dem Herzschrittmacher verbunden ist,

Fig.3 ein Blockschaltbild des erfindungsgemäß durch den Patienten ansteuerbaren Herzschrittmachers sowie

Fig.4 ein Impuls-Zeit-Diagramm, in welchem ein möglicher Zeitablauf der jeweils wirksamen Frequenzen des Herzschrittmachers dargestellt ist.

Zunächst sei anhand des Blockschaltbildes gemäß Fig.3 die Wirkungsweise und Steuerung eines im ganzen mit 11 bezeichneten Herzschrittmachers erläutert:

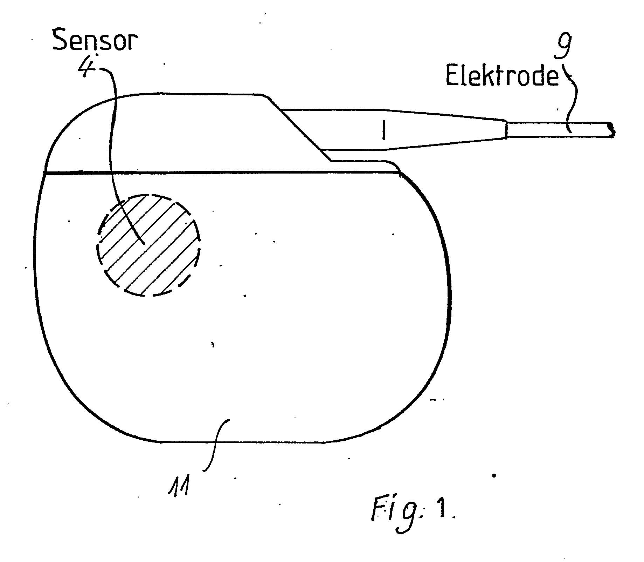

Der implantierbare patientengesteuerte Herzschrittmacher 11 gemäß den Fig.1 u.2 enthält gem. Fig.3 in einem zugleich eine neutrale Elektrode bildenden Gehäuse 10 einen Stimulationsimpulsgenerator 2, der über eine in das Herz des Patienten zu implantierende Stimulationselektrode 9 Stimulationsimpulse mit vom Patienten zu bestimmender steuerbarer Stimulationsfrequenz abgibt, die in noch zu erläuternder Weise in den Herzschrittmacher einprogrammiert ist.

Das intrakardiale Signal, beispielsweise die R- oder P-Welle, wird in einem an die Stimulationselektrode 9 angeschlossenen Verstärker und Detektor 1 verstärkt und erzeugt beim Auftreten dieser Herzaktionspotentiale an seinem Ausgang jeweils einen Inhibitionsimpuls, der den Stimulationsimpulsgenerator 2 für die Abgabe des Stimulationsimpulses sperrt.

Der Steuerkreis 7 bestimmt die Stimulationsgrundfrequenz des Stimulationsimpulsgenerators 2. Die Amplitude des Stimulationsimpulses wird in einer Ausgangsstufe 3 erzeugt. Die Empfindlichkeit des Verstärkers 1 und die Amplitude des

Stimulationsimpulses werden durch von außen empfangene codierte Hochfrequenzsignale in einem Empfänger 8 bestimmt. Die Frequenzsteuerung erfolgt durch Detektion von außen an ein mechanisch von außen durch die Haut bedienbares Betätigungsorgan 4, also durch übertragene mechanische Erschütterungen.

Diese mechanischen Erschütterungen können durch den Patienten, der mit der Hand auf eine entsprechende Implantationsstelle des Herzschrittmachers 11 klopft, ausgelöst werden. Das durch das Betätigungsorgan 4 aufgenommene und in ein elektrisches Signal gewandelte Signal wird in einem Verstärker 5 verstärkt und in einem Impulsformer 6 zu einem digitalen Signal geformt. Die Ansprechschwelle des Impulsformers 6 wird von außen über ein codiertes Hochfrequenzsignal auf den Empfänger 8 bestimmt. Die Frequenz des Schrittmachers wird durch den Steuerkreis 7 nach dem Empfang des von außen mechanisch ausgelösten Signals geändert.

Die Anstiegzeit, in welcher der Herzschrittmacher 11 die maximale Frequenz erreichen soll, sowie die maximale Frequenz selbst werden von außen über codierte Hochfrequenzsignale über den Empfänger 8 ebenso wie auch das Zeitintervall festgelegt, in welchem der Herzschrittmacher 11 mit der maximalen Frequenz stimuliert, bis er automatisch auf seine Grundfrequenz zurückkehrt.

Der Patient hat es aber in der Hand, jederzeit selbst durch einen erneuten "Klopfimpuls" auf die Schrittmacher-Implantationsstelle und inbesondere das implantierte Betätigungsorgan 4 die hohe Stimulationsfrequenz des Schrittmachers 11 wieder auf die Grundfrequenz zu erniedrigen. Die Abfallzeit, in welcher der Herzschrittmacher 11 seine Grundfrequenz wieder erreicht, wird ebenfalls über ein codiertes Hochfrequenzsignal über den Empfänger 8 bestimmt und eingestellt.

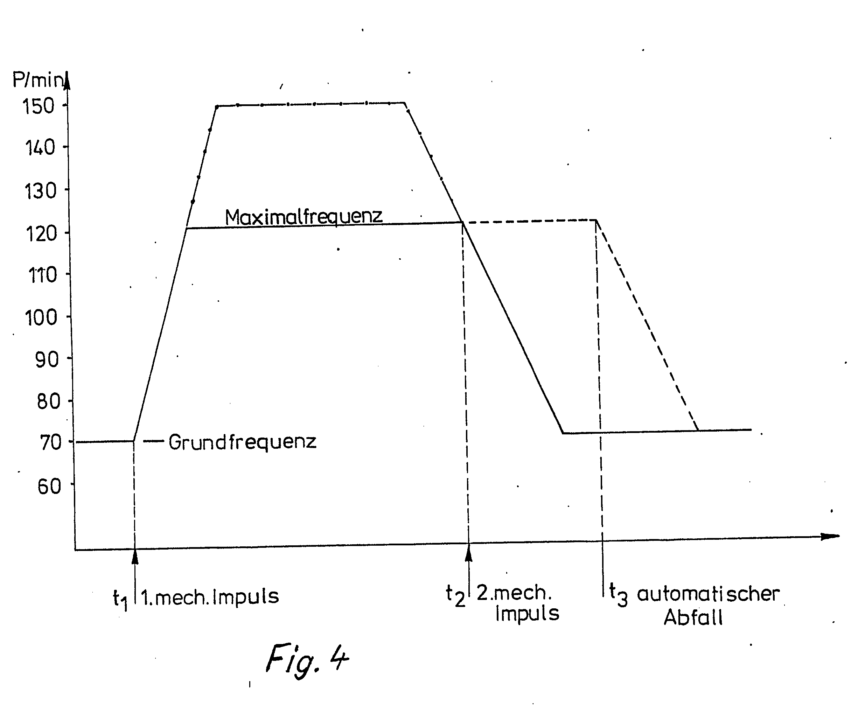

In Fig. 4 ist in einem Impuls-Zeit-Diagramm ein möglicher Zeitablauf dargestellt, wobei bei der Zeit t1 durch den Patienten bewirkt wird, daß die bisherige Grundfrequenz von 70 P/min. über die Anstiegszeit die Maximalfrequenz von z. B. 120 P/min. erreicht wird. Bei der Zeit t2 klopft der Patient wiederum und bewirkt somit, daß nun die Abfallzeit beginnt, bis wieder die Ausgangs- bzw. Grundfrequenz von 70 P/min. erreicht ist. Vergißt der Patient diese Rückschaltung, erfolgt bei der Zeit t3 die automatische Zurückstellung.

Der behandelnde Arzt kann den Herzschrittmacher 11 auf folgende Parameter programmieren:

maximale Stimulationsfrequenz,

Anstiegszeit von Grundfrequenz auf die maximale Stimulationsfrequenz,

Dauer der maximalen Stimulationsfrequenz,

Abfallzeit von der maximalen Stimulationsfrequenz auf die Grundfrequenz.

Sollte der Patient für eine längere Zeit als die Differenz zwischen t1 und t3 die erhöhte Frequenz benötigen, kann er nach dem automatischen Abfall durch einen weiteren mechanischen Impuls den Herzschrittmacher wieder auf die Maximalfrequenz bringen.

Der Herzschrittmacher 11 weist also ein mechanisch von außen durch die Haut bedienbares Betätigungsorgan 4 zur Erhöhung der Herz-Ruhefrequenz und zur Zurückschaltung wieder auf die Herz-Ruhefrequenz auf, so daß eine Anpassung der Stimulationsfrequenz an größere körperliche Leistungen des Herzschrittmacher-Patienten durch diesen selbst ausgelöst werden können.

Gemäß Fig. 1 ist dabei das mechanisch bedienbare Betätigungsorgan 4 auf der der Hautinnenseite zugewandten Seite des Gehäuses 10 des Herzschrittmachers 11 angeordnet.

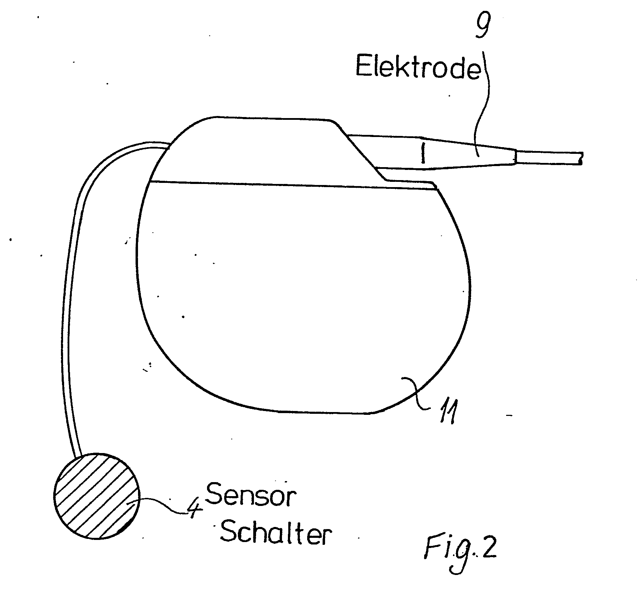

Bei einer abgewandelten Ausführungsform nach Fig.2 ist das mechanisch betätigbare Schaltorgan 4 über eine Leitung 12 mit dem eigentlichen Herzschrittmacher 11 verbunden und unmittelbar unter der Haut, z.B. an einer geschützten Stelle innerhalb der Achselhöhle implantierbar. Das Betätigungsorgan 4 ist dabei, wie vorstehend schon erwähnt, ein klopfempfinglicher Schalter oder Sensor und zum Auslösen oder Betätigen kann bereits ein Klopfimpuls genügen. Eine praxisgerechte Ausführungsform kann dabei darin bestehen, daß der klopfempfindliche Sensor 4 einen piezo-elektrischen Kristall od.dgl. enthält. Der Sensor 4 zur mechanischen Auslösung einer Frequenzänderung des Herzschrittmachers 11 kann zweckmäßigerweise ein Mikrophon sein.

Die Zahl der mechanischen Einwirkungen auf den Sensor 4 zur Ansteuerung der verschiedenen einprogrammierten Frequenzen des Herzschrittmachers 11 kann verschieden sein. Es wurde schon erwähnt, daß eine Einwirkung, d.h. ein einmaliges Klopfen zum Auslösen genügen kann. Vor allem bei mehr als nur einer erhöhten einprogrammierten Frequenz ist es jedoch zweckmäßig, wenn jeder wählbaren Frequenz eine bestimmte Zahl von Klopfimpulsen zugeordnet ist. Dabei können zum Erhöhen der Frequenz ggf. mehr Klopfimpulse auf den Schalter 4 erforderlich sein als zum Vermindern der Frequenz. Beispielsweise kann mit Hilfe zweier Klopfimpulse ausgelöst werden, daß die Stimulationsfrequenz auf 90P/min. steigt. Drei Klopfimpulse können die Frequenz von 110 P/min. auslösen und mit vier Klopfimpulsen könnte eine dritte erhöhte Frequenz von z.B. 140 P/min. ausgelöst werden.

In vielen Fällen werden jedoch eine oder zwei erhöhte Stimulationsfrequenzen genügen.

Insgesamt ergibt sich also ein Herzschrittmacher 11, der zusätzlich zu der Herz-Ruhefrequenz wenigstens eine feste, jedoch über ein auf den Empfänger 8 wirkendes Hochfrequenzsignal einstellbare erhöhte Stimulationsfrequenz oder ggf. mehrere erhöhte Stimulationsfrequenzen aufweist, die durch ein Klopfsignal oder mehrere vorzugsweise unterschiedliche Anzahlen von Klopfimplsen auf das Schaltorgan bzw. den Sensor 4 oder das Mikrophon auslösbar sind. Somit wird dem Patienten selbst die Möglichkeit gegeben, die Stimulationsfrequenz seines Herzschrittmachers 11 an seine jeweils gewünschte Aktivität und Leistung anzupassen und nach vollendeter Leistung auch wieder zurückzuschalten. Aufwendige und komplizierte Fühler, die unter Umständen störanfällig sind und einen erhöhten Herstellungs- und Operationsaufwand bedeuten, werden auf einfache Weise vermieden. Dabei kann dafür gesorgt sein, daß während der Anstiegszeit und/oder der Abfallzeit, die beispielsweise jeweils fünf Sekunden oder eine sonstige vom Arzt vorprogrammierte Zeit dauert, die Klopfimpulse keine Wirkung haben.

Es sei noch erwähnt, daß zum Auslösen der höheren Belastungsfrequenz auch elektromagnetische Felder oder für subkutane Implantationen Lichtimpulse im sichtbaren und unsichtbaren Bereich oder Ultraschallimpulse verwendet werden könnten. Dies hätte jedoch den Nachteil, daß der Patient dann wiederum ein zusätzliches Gerät mit sich tragen müßte, um die Frequenzerhöhung auszulösen. Demgegenüber ist ein solcher zusätzlicher Aufwand bei der mehrfach erwähnten Auslösung über einen oder mehrere Klopfimpulse ebenfalls vermieden.

Ansprüche

/14

DIPL.-ING. H. SCHMITT

DIPL.-ING. W. MAUCHER

- 14 -

DREIKONIGSTR. 1

TELEFON: (0761) 70773

7 0242510

Herr

Dr.Ing.Peter Osypka

Basler Straße 109

7889 Grenzach-Wyhlen

UNSERE AKTE - BITTE STETS ANGEBEN:

E 87 128 MR

## Herzschrittmacher mit einem Steuerteil zur Erhöhung der Herz-Ruhefrequenz

### Ansprüche

1. Herzschrittmacher mit einem Steuerteil zur Erhöhung der Herz-Ruhefrequenz bei erhöhter körperlicher Leistung des Herzschrittmacherpatienten, wobei der Herzschrittmacher (11) ein mechanisch von außen durch die Haut bedienbares Betätigungsorgan (4) zur Erhöhung der Herz-Ruhefrequenz und zur Zurückschaltung auf die Herz-Ruhefrequenz aufweist, dadurch gekennzeichnet, daß als Betätigungsorgan (4) ein klopfempfindlicher Schalter oder Sensor (4) vorgesehen ist und zum Auslösen oder Betätigen wenigstens ein Klopfimpuls erforderlich ist.

2. Herzschrittmacher nach Anspruch 1, dadurch gekennzeichnet, daß der klopfempfindliche Sensor (4) einen piezoelektrischen Kristall od.dgl. enthält.

3. Herzschrittmacher nach Anspruch 1 oder 2, dadurch gekennzeichnet, daß die Zahl der mechanischen Einwirkungen auf den Sensor (4) zur Ansteuerung der verschiedenen einprogrammierten Frequenzen des Herzschrittmachers (11) verschieden ist.

.../15

4. Herzschrittmacher nach Anspruch 3, dadurch gekennzeichnet, daß zum Erhöhen der Frequenz mehr Klopfimpulse auf den Schalter (4) erforderlich sind, als zum Vermindern der Frequenz.

5. Herzschrittmacher nach einem der Ansprüche 1 bis 4, dadurch gekennzeichnet, daß der Sensor (4) zur mechanischen Auslösung einer Frequenzänderung des Herzschrittmachers (11) ein derart unempfindliches Mikrophon ist, daß es nur auf Klopfen reagiert.

6. Herzschrittmacher nach einem der Ansprüche 1 bis 5, dadurch gekennzeichnet, daß das Mikrophon (4) zum Umwandeln des Klopfsignales in ein elektrisches Signal dient und mit einem Verstärker (5) zur Verstärkung dieses Signales verbunden ist, welcher Verstärker (5) zur Umformung dieses elektrischen Signales in ein digitales Signal mit einem Impulsformer (6) verbunden ist.

7. Herzschrittmacher nach einem der Ansprüche 1 bis 6, dadurch gekennzeichnet, daß der Impulsformer (6) eine vorzugsweise von außen über ein codiertes Hochfrequenzsignal od.dgl. auf einen zum Programmieren des Herzschrittmachers dienenden Empfänger (8) einstellbare Ansprechschwelle aufweist.

8. Herzschrittmacher nach einem der Ansprüche 1 bis 7, dadurch gekennzeichnet, daß der Steuerkreis (7) eine vorzugsweise einstellbare Anstiegs- und Abfallzeit für den Übergang von einer Frequenzstufe zu einer anderen des Herzschrittmachers (11) aufweist.

9. Herzschrittmacher nach einem der Ansprüche 1 bis 8, dadurch gekennzeichnet, daß der Steuerkreis (7) bzw. das Herzschrittmacherprogramm eine Höchstzeit für die erhöhte oder maximale Stimulationsfrequenz enthält, die gegebenenfalls über codierte Hochfrequenzsignale über den Programmier-Empfänger (8) des Herzschrittmachers (11) einstellbar ist.

10. Herzschrittmacher nach einem der Ansprüche 1 bis 9, dadurch gekennzeichnet, daß er zusätzlich zu der Herz-Ruhefrequenz wenigstens eine feste, jedoch vorzugsweise über ein auf den Empfänger (8) wirkendes Hochfrequenzsignal einstellbare erhöhte Stimulationsfrequenz, gegebenenfalls mehrere erhöhte Stimulationsfrequenzen aufweist, die durch unterschiedliche Anzahlen von Klopfimpulsen auf das Schaltorgan bzw. den Sensor (4) oder das Mikrophon auslösbar sind.

11. Herzschrittmacher nach einem der Ansprüche 1 bis 10, dadurch gekennzeichnet, daß das Schaltorgan (4) über eine Leitung (12) mit dem eigentlichen Herzschrittmacher (11) verbunden und unmittelbar unter der Haut, z.B. innerhalb der Achselhöhle implantierbar ist.

12. Herzschrittmacher nach einem der Ansprüche 1 bis 10, dadurch gekennzeichnet, daß das Betätigungsorgan (4) auf der der Hautinnenseite zugewandten Seite des Gehäuses (10) des Herzschrittmachers (11) angeordnet ist.

Zusammenfassung

/17

Sensor

4

Elektrode  9

11

Fig. 1.

2/4

9
Elektrode

11

4 Sensor
Schalter

Fig. 2

Fig. 3    Blockschaltbild eines patientengesteuerten Herzschrittmachers

3/4

0242510

Fig. 4

4/4

0242510